# EUROPEAN PATENT SPECIFICATION

(11) **EP 1 942 188 B1**
(45) Date of publication and mention of the grant of the patent: **23.12.2009**
(21) Application number: 07100073.1
(22) Date of filing: 03.01.2007
(51) Int. Cl.: C12N 15/40, C12N 15/42, C12N 15/62, C12N 15/79, C12N 15/82, C12N 15/83, C07K 14/08, C07K 14/09, C12N 5/14, A61K 38/16

(54) **Recombinant viral proteins and particles**
Rekombinante virale Proteine und Partikel
Protéines et particules virales recombinantes

(43) Date of publication of application: 09.07.2008
(73) Proprietor: Academia Sinica, Nan-Kang, Taipei (TW)
(72) Inventor: Liang, Shu-Mei, Nankang, Taipei 11529 (TW); Lin, Na-Sheng, Academia Road, Nankang, Taipei 11529 (TW); Hsu, Yau-Heiu, South District, Taichung 40227 (TW); Liao, Jia-The, 114, TAIPEI (TW)
(74) Representative: Chajmowicz, Marion

(56) References cited:
- WO-A-96/02649
- LIN NA-SHENG ET AL: "Nucleotide sequence of the genomic RNA of bamboo mosaic potexvirus" JOURNAL OF GENERAL VIROLOGY, vol. 75, no. 9, 1994, pages 2513-2518, XP002433228 ISSN: 0022-1317
- ACHARYA R ET AL: "THE THREE-DIMENSIONAL STRUCTURE OF FOOT-AND-MOUTH DISEASE VIRUS AT 2.9 A RESOLUTION" NATURE, NATURE PUBLISHING GROUP, LONDON, GB, vol. 337, no. 6209, 23 February 1989 (1989-02-23), pages 709-716, XP008068624 ISSN: 0028-0836

## Description

### BACKGROUND

Viral infections cause serious damage to the livestock industry. For example, foot-and-mouth disease virus (FMDV) constantly threats domestic livestock throughout the world. FMDV belongs to the aphthovirus genus of the Picornaviridae family. Each FMDV particle contains a single-stranded RNA genome within an icosahedral capsid that consists of 60 copies of each of four proteins, VP1, VP2, VP3 and VP4. While VP1, VP2, and VP3 form the shell of a viral particle, VP4 is located inside the capsid (See Acharya et al., 1989 Nature 337(6209): 709-716). Vaccines are useful to protect livestock against FMDV infection. Conventional FMDV vaccines are based on inactivated virus, the safety of which is not satisfactory. Indeed, FMDV outbreaks occur due to improperly inactivated viruses. Therefore, there is a need for alternative approaches to produce safe FMDV vaccines.

### SUMMARY

This invention relates to use of *Bamboo mosaic virus* (BaMV) particles or proteins as carriers for making immunogenic compositions, such as vaccines. Listed below are the amino acid (aa) sequence of the full-length *Bamboo mosaic virus* coat protein (BaMV CP; SEQ ID NO: 1) and the nucleotide (nt) sequence encoding it (SEQ ID NO: 2):

In one aspect, the invention features an isolated fusion polypeptide containing (i) a carrier fragment that contains the sequence of a BaMV CP or a segment thereof; and (ii) an immunogenic heterologous fragment that is fused to the carrier fragment and at least 3 aa residues in length (e.g., having at least 5, 10, 20, 30, or 37 aa residues). Besides SEQ ID NO: 1, various fragments of SEQ ID NO: 1 can be used as the carrier fragment. Examples include a BaMV CP mutant that lacks the amino terminal 35 aa residues of SEQ ID NO: 1 (SEQ ID NO: 7; underlined above). Preferably, the amino terminus of the carrier fragment is fused to the heterologous fragment. A "heterologous" nucleic acid, gene, or protein is one that originates from a foreign species, or, if from the same species, is substantially modified from its original form.

The aforementioned immunogenic heterologous fragment can contain a sequence of a FMDV VP1 protein or its immunogenic segment. Shown below are the aa sequence of the full-length FMDV VP1 protein (SEQ ID NO: 3) and the nucleotide sequence encoding it (SEQ ID NO: 4):

Examples of the FMDV VP1 immunogenic fragment include TVYNGSSKYGDTSTN NVRGDLQVLAQKAERTLPTSFN (SEQ ID NO: 5), which corresponds to aa 128-164 of SEQ ID NO: 3. Shown below is an exemplary fusion polypeptide, which the sequences of SEQ ID NOs: 5 and 7.

An isolated polypeptide refers to a polypeptide substantially free from naturally associated molecules, i.e., it is at least 75% (i.e., any number between 75% and 100%, inclusive) pure by dry weight. Purity can be measured by any appropriate standard method, e.g., by column chromatography, polyacrylamide gel electrophoresis, or HPLC analysis. An isolated polypeptide of the invention can be purified from a natural source, produced by recombinant DNA techniques, or by chemical methods.

The invention also features an isolated nucleic acid that contains a sequence encoding one of the above-described fusion polypeptides. Examples of the nucleic acid include SEQ ID NOs: 2, 4, 6, 8, and 10, which encode SEQ ID NOs: 1, 3, 5, 7, and 9, respectively. The sequences of SEQ ID NOs: 6, 8, and 10 are shown below: The nucleic acid can further contain the sequences of BaMV Open Reading Frames 1-4 (ORFs 1-4, e.g., those in GenBank Accession No: D26017).

A nucleic acid refers to a DNA molecule (e.g., a cDNA or genomic DNA), an RNA molecule (e.g., an mRNA), or a DNA or RNA analog. A DNA or RNA analog can be synthesized from nucleotide analogs. The nucleic acid molecule can be single-stranded or double-stranded, but preferably is double-stranded DNA. An "isolated nucleic acid" is a nucleic acid the structure of which is not identical to that of any naturally occurring nucleic acid or to that of any fragment of a naturally occurring genomic nucleic acid. The term therefore covers, for example, (a) a DNA which has the sequence of part of a naturally occurring genomic DNA molecule but is not flanked by both of the coding sequences that flank that part of the molecule in the genome of the organism in which it naturally occurs; (b) a nucleic acid incorporated into a vector or into the genomic DNA of a prokaryote or eukaryote in a manner such that the resulting molecule is not identical to any naturally occurring vector or genomic DNA; (c) a separate molecule such as a cDNA, a genomic fragment, a fragment produced by polymerase chain reaction (PCR), or a restriction fragment; and (d) a recombinant nucleotide sequence that is part of a hybrid gene, i.e., a gene encoding a fusion protein. The nucleic acid described above can be used to express the polypeptide of this invention. For this purpose, one can operatively link the nucleic acid to suitable regulatory sequences to generate an expression vector.

A vector refers to a nucleic acid molecule capable of transporting another nucleic acid to which it has been linked. The vector can be capable of autonomous replication or integrate into a host DNA. Examples of the vector include a plasmid, cosmid, or viral vector. The vector of this invention includes a nucleic acid in a form suitable for expression of the nucleic acid in a host cell. Preferably the vector includes one or more regulatory sequences operatively linked to the nucleic acid sequence to be expressed. A "regulatory sequence" includes promoters, enhancers, and other expression control elements (e.g., cauliflower mosaic virus 35S promoter sequences or polyadenylation signals). Regulatory sequences include those that direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vector can be introduced into host cells to produce the polypeptide or viral particle of this invention.

Also within the scope of this invention is a host cell that contains the above-described nucleic acid. Examples include plant cells, *E. coli* cells, insect cells (e.g., using baculovirus expression vectors), yeast cells, or mammalian cells. See e.g., Goeddel, (1990) Gene Expression Technology: Methods in Enzymology 185, Academic Press, San Diego, CA. Preferably, the host cell is a cell of a plant, such a leaf cell of *Chenopodium quinoa* or *Nicotiana benthamiana.*

To produce a polypeptide of this invention, one can culture a host cell in a medium under conditions permitting expression of the polypeptide encoded by a nucleic acid of this invention, and purify the polypeptide from the cultured cell or the medium of the cell. Alternatively, the nucleic acid of this invention can be transcribed and translated *in vitro,* for example, using T7 promoter regulatory sequences and T7 polymerase.

In a further aspect, the invention features a chimeric *Bamboo mosaic virus* particle containing one of the above-described fusion polypeptides. To make this chimeric *Bamboo mosaic virus* particle, one can contact a leaf of a host plant with the above-described nucleic acid, which has the sequences of *Bamboo mosaic virus* Open Reading Frames 1-4; maintain the leaf under conditions permitting formation of a virus particle having the polypeptides encoded by the nucleic acid, and purify the virus particle from the leaf

A fusion polypeptide or chimeric *Bamboo mosaic virus* particle of this invention can also be used to generate specific antibodies that bind specifically to a polypeptide of interest, e.g., FMDV VP1. Accordingly, within the scope ofthis invention is an immunogenic composition including the fusion polypeptide or the chimeric *Bamboo mosaic virus* particle. To inducing an immune response in a subject, one can administer to a subject in need thereof an effective amount of the fusion polypeptide or the chimeric *Bamboo mosaic virus* particle. The subject can be a human or a non-human animal, such as swine, cattle, sheep, or goat.

The details of one or more embodiments of the invention are set forth in the accompanying description below. Other advantages, features, and objects of the invention will be apparent from the detailed description and the claims.

### DETAILED DESCRIPTION

This invention is based, at least in part, on the discovery that a BaMV CP or a BaMV particle can be used as a carrier for generating in a subject an immune response to a polypeptide of interest.

The prior art document WO96/02649 shows particles of the plant virus cowpea mosaic virus (CPMV) containing a foreign peptide inserted in the coat protein. The foreign peptide can be an animal virus antigen derived from foot and mouth disease virus (FMDV) and the viral particles can be used as pharmaceutical.

BaMV, a member of flexuous rod-shaped plant virus of the Potexvirus group, infects both monocotyledonous and dicotyledonous plants (Lin et al., Phytopathology 1992;82:73 1-4). Its genome consists of a single-stranded positive-sense RNA molecule having a 5' cap structure and 3' poly (A) tail. It contains five major open reading frames (ORFs) encoding different proteins for viral replication, movement, and assembly (Lin et al., J. Gen. Virol. 1994;75:2513-2518). Among them, ORF5 encodes a coat protein (CP), which is involved in virus encapsidation and cell-to-cell and long distance movement (Lin et al., Phytopathology 1992;82:731-4 and Lin et al., J. Gen. Virol. 1994;75:2513-8).

Within the scope of this invention is a fusion polypeptide that has an immunogenic polypeptide of interest fused to a carrier containing SEQ ID NO: 1 or its functional equivalent, such as the corresponding sequences from the CP proteins of other BaMV strains.

A functional equivalent of SEQ ID NO: 1 refers to a polypeptide derived from SEQ ID NO: 1, e.g., a fusion polypeptide or a polypeptide having one or more point mutations, insertions, deletions, truncations, or a combination thereof All of the functional equivalents have substantially the activity of BaMV encapsidation and do not affect viral replication. This activity can be determined by a standard assay similar to that described in the examples below.

In particular, such functional equivalents include polypeptides, whose sequences differ from SEQ ID NO: 1 by one or more conservative amino acid substitutions or by one or more non-conservative amino acid substitutions, deletions, or insertions. The following table lists suitable amino acid substitutions:

**TABLE 1. CONSERVATIVE AMINO ACID REPLACEMENTS**

| For Amino Acid | Code | Replace with anv of |
|---|---|---|
| Alanine | A | Gly, Ala, Cys |
| Arginine | R | Lys, Met, Ile, |
| Asparagine | N | Asp, Glu, Gln, |
| Aspartic Acid | D | Asn, Glu, Gln |
| Cysteine | C | Met, Thr |
| Glutamine | Q | Asn, Glu, Asp |
| Glutamic Acid | E | Asp, Asn, Gln |
| Glycine | G | Ala, Pro, |
| Isoleucine | I | Val, Leu, Met |
| Leucine | L | Val, Leu, Met |
| Lysine | K | Arg, Met, Ile |
| Methionine | M | Ile, Leu, Val |
| Phenylalanine | F | Tyr, His, Trp |
| Proline | P | |
| Serine | S | Thr, Met, Cys |
| Threonine | T | Ser, Met, Val |
| Tyrosine | Y | Phe, His |
| Valine | V | Leu, Ile, Met |

In general, a functional equivalent of SEQ ID NO: 1 is at least 50% identical, e.g., at least 60%, 70%, 80%, 90%, or 95% identical, to SEQ ID NO: 1. The "percent identity" of two amino acid sequences or of two nucleic acids is determined using the algorithm of Karlin and Altschul Proc. Natl. Acad. Sci. USA 87:2264-68, 1990, modified as in Karlin and Altschul Proc. Natl. Acad. Sci. USA 90:5873-77, 1993. Such an algorithm is incorporated into the NBLAST and XBLAST programs (version 2.0) of Altschul, et al. J. Mol. Biol. 215:403-10, 1990. BLAST nucleotide searches can be performed with the NBLAST program, score=100, wordlength-12 to obtain nucleotide sequences homologous to the nucleic acid molecules of the invention. BLAST protein searches can be performed with the XBLAST program, score=50, wordlength=3 to obtain amino acid sequences homologous to the protein molecules of the invention. Where gaps exist between two sequences, Gapped BLAST can be utilized as described in Altschul et al., Nucleic Acids Res. 25(17):3389-3402, 1997. When utilizing BLAST and Gapped BLAST programs, the default parameters of the respective programs (e.g., XBLAST and NBLAST) can be used.

A fusion polypeptide of the invention can be obtained as a synthetic polypeptide or a recombinant polypeptide. To prepare a recombinant polypeptide, a nucleic acid encoding it can be linked to another nucleic acid encoding a fusion partner, e.g., Glutathione-S-Transferase (GST), 6x-His epitope tag, or M 13 Gene 3 protein. The resultant fusion nucleic acid expresses in suitable host cells a fusion protein that can be isolated by methods known in the art. The isolated fusion protein can be further treated, e.g., by enzymatic digestion, to remove the fusion partner and obtain the recombinant polypeptide of this invention.

Also within the scope of this invention is a recombinant chimeric *Bamboo mosaic virus* particle that contains the above-described fusion polypeptide. The particle either contains or is free of a wild type BaMV CP. To prepare such a particle, one can introduce a nucleic acid encoding one of the above-described fusion polypeptides and sequences of *Bamboo mosaic virus* ORFs 1-4 and 5' and 3' UTR into a suitable host plant cell and produce a virus particle in the manner described in the examples below. In one embodiment, the fusion polypeptide contains the sequence of SEQ ID NO: 1 or 7.

A fusion polypeptide or chimeric *Bamboo mosaic virus* particle of the invention can be used to generate antibodies in animals (for production of antibodies or treatment of diseases) or humans (for treatment of diseases). Methods of making monoclonal and polyclonal antibodies and fragments thereof in animals are known in the art. See, e.g., Harlow and Lane, (1988) Antibodies: A Laboratory Manual, Cold Spring Harbor Laboratory, New York. The term "antibody" includes intact molecules as well as fragments thereof, such as Fab, F(ab')₂, Fv, scFv (single chain antibody), and dAb (domain antibody; Ward, et. al. (1989) Nature, 341, 544).

In general, to produce antibodies against a protein of interest, one can generate a fusion polypeptide that includes an immunogenic fragment of the protein or a chimeric *Bamboo mosaic virus* particle having the fusion polypeptide. He then mixes the fusion polypeptide or chimeric *Bamboo mosaic virus* particle with an adjuvant and injected the mixture into a host animal. Antibodies produced in the animal can then be purified by peptide affinity chromatography. Commonly employed host animals include rabbits, mice, guinea pigs, and rats. Various adjuvants that can be used to increase the immunological response depend on the host species and include MONTANIDE ISA 206 adjuvant, Freund's adjuvant (complete and incomplete), mineral gels such as aluminum hydroxide, CpG, surface-active substances such as lysolecithin, pluronic polyols, polyanions, peptides, oil emulsions, keyhole limpet hemocyanin, and dinitrophenol. Useful human adjuvants include BCG (bacille Calmette-Guerin) and Corynebacterium parvum.

Polyclonal antibodies, heterogeneous populations of antibody molecules, are present in the sera of the immunized subjects. Monoclonal antibodies, homogeneous populations of antibodies to a polypeptide of this invention, can be prepared using standard hybridoma technology (see, for example, Kohler et al. (1975) Nature 256, 495; Kohler et al. (1976) Eur. J. Immunol. 6, 511; Kohler et al. (1976) Eur. J. Immunol. 6, 292; and Hammerling et al. (1981) Monoclonal Antibodies and T Cell Hybridomas, Elsevier, N.Y.). In particular, monoclonal antibodies can be obtained by any technique that provides for the production of antibody molecules by continuous cell lines in culture such as described in Kohler et al. (1975) Nature 256, 495 and U.S. Patent No. 4,376,110; the human B-cell hybridoma technique (Kosbor et al. (1983) Immunol Today 4, 72; Cole et al. (1983) Proc. Natl. Acad. Sci. USA 80, 2026, and the EBV-hybridoma technique (Cole et al. (1983) Monoclonal Antibodies and Cancer Therapy, Alan R. Liss, Inc., pp. 77-96). Such antibodies can be of any immunoglobulin class including IgG, IgM, IgE, IgA, IgD, and any subclass thereof. The hybridoma producing the monoclonal antibodies of the invention may be cultivated *in vitro* or *in vivo.* The ability to produce high titers of monoclonal antibodies *in vivo* makes it a particularly useful method of production.

A fusion polypeptide or chimeric *Bamboo mosaic virus* particle of the invention can also be used to prepare an immunogenic composition (e.g., a vaccine) for generating antibodies against virus (e.g., FMDV) in a subject susceptible to the virus. Such compositions can be prepared, e.g., according to the method described in the examples below, or by any other equivalent methods known in the art. The composition contains an effective amount of a fusion polypeptide or chimeric *Bamboo mosaic virus* particle of the invention, and a pharmaceutically acceptable carrier such as phosphate buffered saline or a bicarbonate solution. The carrier is selected on the basis of the mode and route of administration, and standard pharmaceutical practice. Suitable pharmaceutical carriers and diluents, as well as pharmaceutical necessities for their use, are described in Remington's Pharmaceutical Sciences. An adjuvant, e.g., a cholera toxin, *Escherichia coli* heat-labile enterotoxin (LT), liposome, immune-stimulating complex (ISCOM), or immunostimulatory sequences oligodeoxynucleotides (ISS-ODN), can also be included in a composition of the invention, if necessary. The fusion polypeptide, fragments or analogs thereof or chimeric *Bamboo mosaic virus* particle may be components of a multivalent composition of vaccine against various diseases.

Methods for preparing vaccines are generally well known in the art, as exemplified by U.S. Patents. 4,601,903; 4,599,231; 4,599,230; and 4,596,792. Vaccines may be prepared as injectables, as liquid solutions or emulsions. A fusion polypeptide or chimeric *Bamboo mosaic virus* particle of this invention may be mixed with physiologically acceptable and excipients compatible. Excipients may include, water, saline, dextrose, glycerol, ethanol, and combinations thereof The vaccine may further contain minor amounts of auxiliary substances such as wetting or emulsifying agents, pH buffering agents, or adjuvants to enhance the effectiveness of the vaccines. Methods of achieving adjuvant effect for the vaccine include use of agents, such as aluminum hydroxide or phosphate (alum), commonly used as 0.05 to 0.1 percent solutions in phosphate buffered saline. Vaccines may be administered parenterally, by injection subcutaneously or intramuscularly. Alternatively, other modes of administration including suppositories and oral formulations may be desirable. For suppositories, binders and carriers may include, for example, polyalkalene glycols or triglycerides. Oral formulations may include normally employed incipients such as, for example, pharmaceutical grades of saccharine, cellulose, magnesium carbonate and the like. These compositions take the form of solutions, suspensions, tablets, pills, capsules, sustained release formulations or powders and contain 10-95% of the fusion polypeptide or chimeric *Bamboo mosaic virus* particle.

The vaccines are administered in a manner compatible with the dosage formulation, and in an amount that is therapeutically effective, protective and immunogenic. The quantity to be administered depends on the subject to be treated, including, for example, the capacity of the individual's immune system to synthesize antibodies, and if needed, to produce a cell-mediated immune response. Precise amounts of active ingredient required to be administered depend on the judgment of the practitioner. However, suitable dosage ranges are readily determinable by one skilled in the art and may be of the order of micrograms of the polypeptide of this invention. Suitable regimes for initial administration and booster doses are also variable, but may include an initial administration followed by subsequent administrations. The dosage of the vaccine may also depend on the route of administration and varies according to the size of the host.

As described in the examples below, the above-described fusion polypeptide or chimeric *Bamboo mosaic virus* particle can be used to induce immune response in a subject against virus infection, such as FMDV infection. A subject susceptible to FMDV infection can be identified and administered a composition of the invention. The dose of the composition depends, for example, on the particular polypeptide/virus particle, whether an adjuvant is co-administered, the type of adjuvant co-administered, the mode and frequency of administration, as can be determined by one skilled in the art. Administration is repeated as necessary, as can be determined by one skilled in the art. For example, a priming dose can be followed by three booster doses at weekly intervals. A booster shot can be given at 4 to 8 weeks after the first immunization, and a second booster can be given at 8 to 12 weeks, using the same formulation. Sera or T-cells can be taken from the subject for testing the immune response elicited by the composition against the FMDV. Methods of assaying antibodies or cytotoxic T cells against a protein or infection are well known in the art. Additional boosters can be given as needed. By varying the amount of polypeptide/virus particle, the dose of the composition, and frequency of administration, the immunization protocol can be optimized for eliciting a maximal immune response. Before a large scale administering, efficacy testing is desirable. In an efficacy testing, a non-human subject can be administered via an oral or parenteral route with a composition of the invention. After the initial administration or after optional booster administration, both the test subject and the control subject (receiving mock administration) can be challenged with, e.g., 0.5 mL of 1 × 10⁵ TCID₅₀ of FMDV O/ Taiwan/ 97 by subcutaneous injection. The subjects are then monitored for signs of FMD for 14 days. Signs of FMD include elevation of body temperatures above 40°C for three successive days, lameness, vesicular lesions around the mouth, and coronary bands on the leg.

The above-described BaMV CP polypeptide can be used as a carrier and linked to other antigens of interest to generate antibodies against the antigens. The polypeptides fragment can be generally utilized to prepare chimeric molecules and conjugate compositions against pathogenic bacteria, including encapsulated bacteria.

The specific examples below are to be construed as merely illustrative, and not limitative of the remainder of the disclosure in any way whatsoever. Without further elaboration, it is believed that one skilled in the art can, based on the description herein, utilize the present invention to its fullest extent.

### Example 1

In this example, a VP1 antigenic epitope was fused to the amino terminus of a truncated coat protein of BaMV to generate a fusion protein. A chimeric BaMV having the fusion protein was also produced.

The full-length infectious cDNA of BaMV-S with an upstream cauliflower mosaic virus 35S promoter sequence was cloned in plasmid pUC119 in the manner described in Lin et al,. J. Gen. Virol. 2004;85:251-9. Vector pBS-d35CP was derived from aforementioned BaMV cDNA clone, in which the sequence encoding the N-terminal 35 amino acid sequence of CP was deleted and multiple cloning sites (*Age*I-*Nlre*I-*Not*I) were inserted by a polymerase chain reaction (PCR)-based method. A sequence encoding amino acids 128-164 of FMDV VP 1 (O/ Taiwan/ 97) was inserted into pBS-d35CP by PCR using plasmid pVP1/Q15 as a template (Wang et al., Vaccine 2003; 21:3721-9.). The primers used were pr128164N (5'-GGgctagcAccatgg-ACACCGTCTACAACGGGAG-3' (SEQ ID NO:11); the sequences in small case represent *Nhe*I and *Nco*I sites; the *Nco*I recognition sequence provides an AUG initiation codon) and pr128164C (5'-TTgcggccgcGTTGAAGGAGGTAGGC-3'(SEQ ID NO:12); the sequence in small case represents a *Not*I site). The PCR reaction was carried out at an initial temperature of 94°C for 5 minutes followed by 25 cycles of 94°C for 30 seconds, 50°C for 30 seconds, and an extension at 72°C for 30 seconds. The amplified fragment corresponding to VP1 epitope was purified and cloned into plasmid pBS-d35CP at *Nhe*I and *Not*I sites to generate a plasmid denoted as pBVP1. This vector encodes the genome of a chimeric BaMV, namely BVP 1.

To examine the infectivity of this chimeric virus in plants, both systemic host *Nicotiana benthamiana* (*N. benthamiana*) and local lesion host *Chenopodium quinoa* (*C. quinoa*) were tested.

Local lesion host *C. quinoa* and systemic host *N. benthamiana* plants were grown in a greenhouse exposed to normal daylight. For the infectivity assay, approximately 1 µg of purified pBVP1 DNA in a 10 µl volume of double-distilled H₂O was used to inoculate each leaf of test plants at the 6-leaf stage (Lin et al., J. Gen. Virol. 2004 ;85:251-9). Observation for local lesions was made 10 days post-inoculation. For antigen preparation, *C. quinoa* plants with eight to ten fully expanded leaves were inoculated with sap extracted from *C. quinoa* plants infected with BaMV-S or BVP1. Inoculated leaves were harvested at 10 days post-inoculation. Virions were subsequently purified from fresh leaves in the manner described in Lin et al., Phytopathology, 1992; 82:731-4. The yield of purified virus was determined by ultraviolet absorption, assuming an absorbance (0.1%, 260 nm) of 3.0. The amount of VP1 epitope expressed in the chimeric virus BVP1 is estimated to be about 14.3% of the total virus weight. Purified virions were dissolved in BE buffer (10 mM Borate, pH 9.0, 1mM EDTA), and then stored at -20°C for immunization of swine.

It was found that, in *N. benthamiana*, the BVP1-infected plants showed milder mosaic symptoms than BaMV-S-infected plants. In *C. quinoa*, the chlorotic local lesions formed by BVP1 were distinct from necrotic local lesions caused by wild-type BaMV-S. Nevertheless, the results demonstrate that the chimeric mutant virus undoubtedly possessed the ability to infect healthy *N. benthamiana* or *C. quinoa* plants.

### Example 2

The above-mentioned chimeric virus BVP 1 was examined to determine whether it could express the VP1 epitope. Total protein samples taken from *C. quinoa* leaves inoculated with mock, wild-type BaMV-S, BS-d35CP and BVP1 were subjected to SDS-polyacrylamide gel electrophoresis.

It was found that chimeric CP from BVP1 migrated more slowly than wild-type CP or N-terminal truncated CP, while no CP was found in mock-inoculated leaves. The result indicates an apparent size difference between the wild-type of and chimeric BVP1 CP and the fusion of the VP1 epitope to the CP of BVP1.

To further confirm that CP of BVP1 contains a VP1 epitope, Western blotting assay was performed using a rabbit against FMDV VP1 serum or a serum from FMDV infected swine. The rabbit anti-FMDV VP 1 serum was prepared in the manner described in Wang et al., 2003, Vaccine 2003; 21:3721-9. The serum from an FMDV pig was obtained from the Animal Health Research Institute, COA, R.O.C. Total proteins were prepared from mock- or virus-inoculated *C. quinoa* with an 1:2 (w/v) extraction buffer (50 mM Tris-HCl, pH 8.0, 10 mM KC1, 10 mM MgC12, 1 mM EDTA, 20% glycerol and 2% SDS) and heated at 100°C for 5 minutes. The Protein samples were then separated by 12% SDS-polyacrylamide gel electrophoresis and electrophoretically transferred to Immobilon-P membranes (Bio-Rad) with 200 mA for 1 hour at 4°C. After blocking, the membranes were probed with anti-BaMV-S CP or anti-FMDV VP1 antibodies and then processed in the manner described in Lin et al., Phytopathology 1992; 82:731-4 and Lin et al., J. Gen. Virol. 2004; 85:251-9.

It was found that that both sera recognized the chimeric CP corresponding to a protein band with expected 31 kDa size. In contrast, no VP1 protein band was detected in the wild-type CP of BaMV-S or truncated CP of BS-d35CP. Additional immunoblotting studies showed that even after five subsequent passages in *C. quinoa*, the CP of BVP1 extracted from *C. quinoa* leaves still contained a major protein band of a similar mobility. Taken together, these results demonstrate that the chimeric virus BVP1 is able to stably express the VP1 epitope of FMDV as a fusion protein in its CP.

### Example 3

To above-described BVP1 chimeric virus was tested for its ability to induce specific antibodies in swine.

The chimeric BVP1 virus particles were isolated from BVP1 infected *C. quinoa* leaf tissue. The yield of the purified virus was about 0.2-0.5 mg per gram of fresh leaf tissue. Specific pathogen-free (SPF) female or castrated male swine (2-month-old, weighing approximately 25 kg) were obtained in Taiwan. Two groups of SPF swine (three in each group) were immunized with 5 mg and 10 mg of the BVP1 chimeric virus preparation respectively by intramuscular immunization. More specifically, they were injected into the neck muscles beside the ears with 10 mg or 5 mg of BVP1 virions emulsified with equal volumes of Montanide ISA 206 adjuvant (Seppic, France). In addition, two swine were injected with wild-type virus BaMV-S and another two swine with sterile phosphate-buffered saline (PBS; 140 mM NaCl, 2.7 mM KC1, 10 mM Na₂HPO4, 1.8 mM KH₂PO4, pH 7.3) buffer as negative control groups.

Six weeks after priming, booster injections at the same dosages were given to all swine. Sera were collected for analysis from the immunized swine at days 0, 28, 42, 56, and 63. Three weeks after the booster, all swine were challenged with 0.5 mL of 1 × 10⁵ TCID₅₀ of FMDV O/ Taiwan/ 97 by injection into the right front heel bulb. The swine were then monitored for signs of FMD for 14 days. Signs of FMD included elevation of body temperatures above 40°C for three successive days, lameness, vesicular lesions on the snout, and coronary bands on the legs (Wang et al., Vaccine 2003;21:3721-9).

To examine immunization effects, ELISA was performed in the manner with minor modifications as described in (Shieh et al., Vaccine 2001; 19:4002-10). In brief, after the formation of plate-bound antigen-antibody complexes, the plates were washed three times with PBS containing 0.1 % Tween-20 (PBST), and incubated with biotinylated goat anti-swine IgG antibodies for 1 hour at 37°C. The plates were subsequently washed and streptavidin: peroxidase (1: 3000 dilutions) added. After incubation for 1 hour at room temperature, the plates were washed again. Enzyme substrate 3, 3', 5, 5'-tetramethylbenzidine (Sigma) was then added, and the reaction carried out at room temperature for 10 minutes. Finally, an equal volume of 1 N H₂SO₄ was added to stop the reaction and the absorbance at 450 nm was measured by an ELISA reader.

ELISA showed that four weeks after priming, the immunized swine elicited significant titers of anti-VP1 antibodies (880±434 for the 5 mg group and 2382±1098 for the 10 mg group). The level of the specific anti-VP1 antibodies increased and reached a plateau two weeks later. In contrast, the swine injected with wild type virus BaMV-S or PBS buffer showed little anti-VP 1 antibodies in ELISA. This result demonstrated that the chimeric BVP1 virus induces specific anti-VP1 antibodies in swine.

Presence of neutralizing antibodies (NA) in the sera of BVP1 immunized swine were confirm according the method described in Shieh et al., Vaccine 2001; 19:4002-10. In brief, sera from test animals were inactivated at 56°C for 30 minutes. Each serum sample or control serum (50 µl) was added to the well at the end of each row of a 96-well tissue culture plate, and then diluted in a two-fold serial dilution across the plates. Fifty microliters of a 100 TCID₅₀ virus suspension were added to each well, and then the plate was vortexed for 1 minute. After incubation at 37°C in 5% CO₂ for 90 minutes, 100 µl of BHK-21 cell suspension (10⁶ cells/ ml) in Eagle's MEM containing 3% fetal bovine serum (FBS) was added to each well, and incubated for 48 hours. The cells were then observed under microscope. Disruption of cell monolayer and change of cells from spindle to round shape in the wells indicated the presence of cytopathic effect of the virus.

Titers were determined after 48 hours of incubation at 37°C in a water-saturated atmosphere with 5% CO₂ and expressed as the reciprocal of the final dilution of serum that neutralizes the cytopathic effect of the virus at the 50% end-point. The results are summarized in Table 2 below:

**Table 2 Neutralizing Antibody (NA) Titers in Swine Immunized with BVP1**

| | NA titer (individuals) ^{b} | | | |
|---|---|---|---|---|
| Antigen ^{a} | n | 4 WPP^{c} | 6 WPP | 9 WPP |
| BVP1 10 mg | 3 | 57 (6, 91, 64) | 217 (32, 362, 256) | 264 (23, 512, 256) |
| BVP1 5 mg | 3 | 12 (8, 11, 16) | 227 (64, 364, 256) | 210 (11, 362, 256) |
| BaMV-S | 2 | - (-, -) | - (-, -) | - (-, -) |
| Negative control ² | | - (-, -) | - (-, -) | - (-, -) |

| | | | | |
|---|---|---|---|---|
| ^{a} Swine were inoculated with two intramuscular injections of 10 mg or 5 mg of BVP1 or 5 mg of BaMV-S virions emulsified in an equal amount of ISA206 adjuvant at a 6-week interval. Swine immunized with PBS buffer were used as a negative control group. ^{b} The titer ofNA from each pig expressed as the reciprocal of the final dilution of the serum that caused a 50 % reduction in the test virus activity as described in the materials and methods. (-) indicates that no NA was detected. ^{c} WPP: weeks post priming. | | | | |

As shown in Table 2, swine immunized with either wild-type BaMV-S virus or PBS buffer did not produce any NA against FMDV. In contrast, swine immunized with 5 mg or 10 mg of BVP1 produced NA four weeks post priming. The titers increased even further 6 weeks post priming. Boosting with similar amount of BVP1 at 6 weeks post priming, however, did not significantly increase the NA. This result demonstrated that inoculation of swine with BVP1 virus once is sufficient to induce high level ofNA against FMDV.

### Example 4

The ability of peripheral blood mononuclear cells (PBMCs) to produce IFN-γ was analyzed in the above-described swine to evaluate whether BVP1 immunization could induce a cell-mediated immune response in addition to a humoral response. IFN-γ is a cytokine that plays important role in cell-mediated immune responses.

Specifically, PBMCs were isolated from the test swine and seeded in triplicate in 6-well culture plates at a concentration of 1 × 10⁷ cells per well in 2 mL of DMEM culture medium supplemented with 10% FBS and 1% penicillin and streptomycin. After overnight culture, the cells were incubated with 5 µg/mL of recombinant VP 1 (rVP1) or 1 µg/mL ofphytohemagglutinins (PHA, Sigma) for 6 hours at 37°C in 5% CO₂. Cells incubated with PHA were used as positive controls. Following the incubation, the cells were lysed in TRIzol™ reagent (Invitrogen) and total cellular RNA was isolated according to the manufacturer instructions. The concentration of total cellular RNA was quantified by determination of optical density at 260 nm. The total cellular RNA was then reversely transcribed into complementary DNA (cDNA) by SuperScript III^{™} reverse transcriptase (Invitrogen). The resulting cDNA was subjected to real-time PCR.

Total IFN-γ mRNA was quantified by real-time PCR. Real-time PCR was set up using a SYBR Green system in a LightCycler instrument (RocheApplied Science) in a final volume of 20 µL with the FastStart DNA Master SYBR Green I Kit (Roche), including heat-activatable *Taq* polymerase, plus 4 mM MgCl₂, each primer (primer sequences were SW-IFN γ (F4): 5'-GCT CTG GGA AAC TGA ATG ACT TCG (SEQ ID NO:13) and SW-IFN γ (R4): 5'-GAC TTC TCT TCC GCT TTC TTA GGT TAG) (SEQ ID NO: 14) at 0.5 µM and 2 µL of cDNA prepared as described above. Following polymerase activation (95°C for 10 minutes), 40 cycles were run with a 15 seconds denaturing at 95°C, a 2 second annealing at 60°C, and a 15 seconds extension at 72°C. The temperature transition rate was 20°C per second for all steps. The amount of PCR product was measured once every cycle immediately after the 72°C incubation (extension step) by detection of the fluorescence associated with the binding of SYBR Green I to the amplification product. Fluorescence curves were analyzed with the LightCycler software, version 3.0 (RocheApplied Science). The primers were synthesized by Invitrogen Life Technologies. For each sample, the amount of IFN-γ was determined by comparing with a standard curve and normalized by using β-actin as the endogenous reference. All samples were processed in triplicate.

It was found that IFN-γ production by the PBMCs from BVP 1-immunized swine increased by 3 folds upon stimulation by recombinant VP1 (rVP1). In contrast, IFN-γ production by PBMCs from swine immunized with BaMV-S or injected with PBS showed no increase upon rVP1 stimulation. As a control, PBMCs from all swine, once stimulated with PHA, produced similar amounts of IFN-γ. These results demonstrate that the immune cells of BVP1-immunized swine are capable of producing IFN-yupon rVP1 stimulation.

### Example 5

To determine the efficacy of BVP1 immunization, swine inoculated with or without BVP1 were challenged with 1 × 10⁵ TCID₅₀ of FMDV (O/Taiwan/97) three weeks after boosting and then monitored the symptoms of FMD for two weeks. Those without symptoms were determined as protected. The results were summarized in Table 3 below:

**Table 3. Protection of recombinant virus BVP1 in swine against FMDV challenge**

| Group | No. of Challenged | No. of Protected | Protection Rate |
|---|---|---|---|
| BVP1 10 mg | 3 | 3 | 100% |
| BVP1 5 mg | 3 | 3 | 100% |
| BaMV-S | 2 | 0 | 0% |
| Negative control | 2 | 0 | 0% |

As shown in Table 3, swine immunized with 5 mg or 10 mg BVP1 were symptom-free and completely protected during the two week period after challenge. In contrast, swine immunized with BaMV-S or injected with PBS (negative control) showed serious FMD symptoms on the second day after FMDV challenge. This result demonstrated that chimeric virus BVP1 is useful for vaccinating swine against FMDV.

It was known that immunization of swine with *E. coli*-derived rVP1 was effective in protecting the swine against FMDV challenge. The efficacies of BVP1 and similar amounts *E. coli*-derived rVP 1 were compared. More specifically, swine were inoculated with two intramuscular injections of 8 mg or 4 mg of rVP1 emulsified in an equal volume of ISA206 adjuvant at a 6-week interval in the manner described in Wang et al., Vaccine 2003;21:3721-9. Effects of rVP1 in eliciting neutralizing antibodies (NA) and protection against FMDV were evaluated in the same manner describe above. The results were summarized in Table 4 below:

**Table 4. Effects of rVP1 immunization**

| | NA titer (individuals)^{a} | | | Protection |
|---|---|---|---|---|
| Antigen | n 3 WPP | 6 WPP | 10 WPP | |
| 8 mg rVP1 | 3 13 11,11, 16) | 22(11,11,45) | 21 (-, 64, -) | 100% |
| 4 mg rVP1 | 3 11 (11,11, 11) | 4 (11, -, -) | 75 (181, 45, -) | 100% |
| Negative control | 2 6 ( -, 11) | - (- , -) | - (- , -) | 0 % |

| | | | | |
|---|---|---|---|---|
| a: The titer ofNA from each pig is expressed as the reciprocal of the final dilution of the serum that caused a 50 % reduction in the test virus activity as described in the materials and methods section. (-) denotes absence ofNA. | | | | |

As shown in Table 4, rVP 1 led to full protection in all immunized swine. Nonetheless, it generated NA in some but not all immunized swine. Also, the average NA elicited by rVP1 was not as high as those of BVP1 immunization (see Tables 2 and 4). The results suggest that BVP1 expressing a 37-aa VP1 fragment is more effective than *E.coli*-derived full-length rVP1 in eliciting humoral immune responses.

Previously, recombinant *E. coli* derived VP1 (rVP1), a 26 kDa polypeptide, were purified. It could induce protective immunity in swine. Comparing the composition described above, rVP1 elicited neutralizing antibodies in some but not all immunized swine. The average titer of neutralizing antibodies elicited by rVP 1 was not as high as those of BVP1 (Tables 2 and 4). It was unexpected that BVP1, containing only 37 amino acids of VP1 fused to BaMV CP, led to better neutralizing antibody responses than rVP1. It was also unexpected that BVP 1 CP mutant lacking up to N terminal 35 amino acid residues could form virus that were still able to infect both systemic host (*N. benthamiana*) and local lesion host (*C. quinoa*) plants.

The above results indicate that the BaMV expression vector system and the chimeric virus described herein are effective for generating neutralizing antibodies, due to the flexibility of BaMV in accommodating foreign peptides and expressing them effectively. Moreover, BaMV has additional advantages over other plant virus. For example, as no natural transmitting vector for BaMV has been found, BaMV is safer (Lin et al. Phytopathology 1992;82:731-4). In contrast, many plant viruses are pathogens for most crops. Also, BaMV, as a carrier, has a larger capacity to accommodate a longer heterologous polypeptide.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the scope thereof, can make various changes and modifications of the invention to adapt it to various usages and conditions. Thus, other embodiments are also within the scope of the following claims.

## Claims

1. An isolated nucleic acid encoding a fusion polypeptide, wherein the fusion polypeptide includes
a carrier fragment that contains the sequence of a *Bamboo mosaic virus* coat protein or contains a segment thereof; and
an immunogenic heterologous fragment that is fused to the carrier fragment and at least 3 amino acids in length.

2. The nucleic acid of claim 1, wherein the immunogenic heterologous fragment contains the sequence of a foot-and-mouth disease virus VP 1 protein or contains an immunogenic segment thereof.

3. The nucleic acid of claim 2, wherein the immunogenic heterologous fragment contains SEQ ID NO: 3.

4. The nucleic acid of claim 3, wherein the immunogenic segment contains SEQ ID NO: 5.

5. The nucleic acid of claim 1, wherein the heterologous fragment is fused to the amino terminus of the carrier fragment.

6. The nucleic acid of claim 1, wherein the carrier fragment contains SEQ ID NO: 7.

7. The nucleic acid of claim 1, wherein the immunogenic heterologous fragment is at least 37 amino acids in length.

8. The nucleic acid of claim 1, wherein the polypeptide includes the sequence of SEQ ID NO: 9.

9. The nucleic acid of claim 1, wherein the nucleic acid further comprises the sequences of *Bamboo mosaic virus* Open Reading Frames 1-4.

10. An isolated polypeptide encoded by the nucleic acid of claim 1.

11. An expression vector comprising the nucleic acid of claim 1.

12. A host cell comprising the nucleic acid of claim 1.

13. The host cell of claim 12, wherein the host cell is a cell of a plant.

14. The host cell of claim 13, wherein the plant is *Chenopodium quinoa* or *Nicotiana benthamiana*.

15. The host cell of claim 13, wherein the cell is a leaf cell.

16. A method of producing a fusion polypeptide, comprising culturing the cell of claim 12 in a medium under conditions permitting expression of a polypeptide encoded by the nucleic acid, and purifying the polypeptide from the cultured cell or the medium.

17. A chimeric *Bamboo mosaic virus* particle comprising the fusion polypeptide encoded by the nucleic acid of claim 1.

18. A method of producing a chimeric *Bamboo mosaic virus* particle, comprising
providing a nucleic acid of claim 1, the nucleic acid having sequences of *Bamboo mosaic virus* Open Reading Frames 1-4;
contacting the nucleic acid with a leaf of a host plant;
maintaining the leaf under conditions permitting formation of a virus particle having the polypeptides encoded by the nucleic acid, and
purifying the virus particle from the leaf.

19. An immunogenic composition comprising the fusion polypeptide of claim 10.

20. An immunogenic composition comprising the chimeric *Bamboo mosaic virus* particle of claim 17.

21. Use of the fusion polypeptide of claim 10 for the manufacture of a composition for inducing an immune response in a subject.

22. The use of claim 21, wherein the subject is a non-human animal.

23. The use of claim 22, wherein the non-human animal is a pig.

24. Use of the chimeric *Bamboo mosaic virus* particle of claim 17 for the manufacture of a composition for inducing an immune response in a subject.

25. The use of claim 24, wherein the subject is a non-human animal.

26. The use of claim 25, wherein the non-human animal is a pig.

## Patentansprüche

1. Eine isolierte Nukleinsäure, die ein Fusionspolypeptid kodiert, wobei das Fusionspolypeptid folgendes einschließt:
ein Trägerfragment, das die Sequenz eines Bambus-Mosaik-Virus Hüllproteins enthält oder ein Segment davon enthält; und
ein immunogenes heterologes Fragment, das an das Trägerfragment fusioniert ist und mindestens eine Länge von 3 Aminosäuren aufweist.

2. Die Nukleinsäure nach Anspruch 1, wobei das immunogene heterologe Fragment die Sequenz eines Maul- und Klauenseuchen Virus VP1 Proteins enthält oder ein immunogenes Segment davon enthält.

3. Die Nukleinsäure nach Anspruch 2, wobei das immunogene heterologe Fragment SEQ ID NO: 3 enthält.

4. Die Nukleinsäure nach Anspruch 3, wobei das immunogene Segment SEQ ID NO: 5 enthält.

5. Die Nukleinsäure nach Anspruch 1, wobei das heterologe Fragment an den Amino-Terminus des Trägerfragments fusioniert ist.

6. Die Nukleinsäure nach Anspruch 1, wobei das Trägerfragment SEQ ID NO: 7 enthält.

7. Die Nukleinsäure nach Anspruch 1, wobei das immunogene heterologe Fragment mindestens eine Länge von 37 Aminosäuren aufweist.

8. Die Nukleinsäure nach Anspruch 1, wobei das Polypeptid die Sequenz der SEQ ID NO: 9 einschließt.

9. Die Nukleinsäure nach Anspruch 1, wobei die Nukleinsäure ferner die Sequenzen der Bambus-Mosaik-Virus offene Leserrahmen 1-4 umfasst.

10. Ein isoliertes Polypeptid, kodiert durch die Nukleinsäure nach Anspruch 1.

11. Ein Expressionsvektor, umfassend die Nukleinsäure nach Anspruch 1.

12. Eine Wirtszelle, umfassend die Nukleinsäure nach Anspruch 1.

13. Die Wirtszelle nach Anspruch 12, wobei die Wirtszelle eine Zelle einer Pflanze ist.

14. Die Wirtszelle nach Anspruch 13, wobei die Pflanze *Chenopodium quinoa* oder *Nicotiana benthamiana* ist.

15. Die Wirtszelle nach Anspruch 13, wobei die Zelle eine Blattzelle ist.

16. Ein Verfahren zur Herstellung eines Fusionspolypeptids, umfassend Kultivierung der Zellen nach Anspruch 12 in einem Medium unter Bedingungen, die die Expression eines Polypeptids kodiert durch die Nukleinsäure ermöglichen, und Aufreinigung des Polypeptids aus der kultivierten Zelle oder dem Medium.

17. Ein chimärer *Bambus-Mosaik*-Viruspartikel, umfassend das Fusionspolypeptid kodiert durch die Nukleinsäure nach Anspruch 1.

18. Ein Verfahren zur Herstellung eines chimären *Bambus-Mosaik*-Viruspartikels, umfassend
zur Verfügung stellen einer Nukleinsäure nach Anspruch 1, wobei die Nukleinsäure Sequenzen des Bambus-Mosaik-Virus offene Leserahmen 1-4 aufweist;
In Kontakt bringen der Nukleinsäure mit einem Blatt einer Wirtspflanze; Aufrechterhalten der Blätter unter Bedingungen, die die Bildung eines Viruspartikels, der das Polypeptid aufweist, das durch die Nukleinsäure kodiert wird, ermöglichen; und Aufreinigen des Viruspartikels aus dem Blatt.

19. Eine immunogene Zusammensetzung, umfassend das Fusionspolypeptid nach Anspruch 10.

20. Eine immunogene Zusammensetzung, umfassend das chimären Bambus-Mosaik-Viruspartikel nach Anspruch 17.

21. Verwendung des Fusionspolypeptids nach Anspruch 10 zur Herstellung einer Zusammensetzung zum Induzieren einer Immunantwort in einem Subjekt.

22. Die Verwendung nach Anspruch 21, wobei das Subjekt ein nicht-menschliches Tier ist.

23. Die Verwendung nach Anspruch 22, wobei das nicht-menschliche Tier ein Schwein ist.

24. Verwendung eines chimären *Bambus-Mosaik*-Viruspartikels nach Anspruch 17 zur Herstellung einer Zusammensetzung zum Induzieren einer Immunantwort in einem Subjekt.

25. Die Verwendung nach Anspruch 24, wobei das Subjekt ein nicht-menschliches Tier ist.

26. Die Verwendung nach Anspruch 25, wobei das nicht-menschliche Tier ein Schwein ist.

## Revendications

1. Acide nucléique isolé codant pour un polypeptide de fusion, le polypeptide de fusion incluant
un fragment support qui contient la séquence d'une protéine d'enveloppe du virus *Bamboo mosaic* ou qui contient un segment de celle-ci ; et
un fragment immunogène hétérologue qui est fusionné au fragment support et a au moins 3 acides aminés de longueur.

2. Acide nucléique selon la revendication 1, où le fragment hétérologue immunogène contient la séquence d'une protéine VP1 du virus de la maladie de la fièvre aphteuse, ou qui contient un segment immunogène de celle-ci.

3. Acide nucléique selon la revendication 2, où le fragment hétérologue immunogène contient la SEQ ID NO :3.

4. Acide nucléique de la revendication 3, où le segment immunogène contient la SEQ ID NO: 5.

5. Acide nucléique de la revendication 1, où le fragment hétérologue est fusionné à l'extrémité amino terminale du fragment support.

6. Acide nucléique de la revendication 1, où le fragment support contient la SEQ ID NO :7.

7. Acide nucléique de la revendication 1, où le fragment hétérologue immunogène a au moins 37 acides aminés de longueur.

8. Acide nucléique de la revendication 1, où le polypeptide inclut la séquence de la SEQ ID NO :9.

9. Acide nucléique de la revendication 1, où l'acide nucléique comprend en outre les séquences des cadres de lecture ouverte 1-4 du virus *Bamboo mosai'c.*

10. Polypeptide isolé codé par l'acide nucléique de la revendication 1.

11. Vecteur d'expression comprenant l'acide nucléique de la revendication 1.

12. Cellule hôte comprenant l'acide nucléique de la revendication 1.

13. Cellule hôte de la revendication 12, où la cellule hôte est une cellule de plante.

14. Cellule hôte de la revendication 13, où la plante est *Chenopodium quinoa* ou *Nicotiana benthamiana.*

15. Cellule hôte de la revendication 13, où la cellule est une cellule de feuille.

16. Procédé de production d'un polypeptide de fusion, comprenant la culture de la cellule de la revendication 12 dans un milieu dans des conditions permettant l'expression d'un polypeptide codé par l'acide nucléique, et la purification du polypeptide à partir de la cellule cultivée ou du milieu.

17. Particule chimérique de virus *Bamboo mosai'c,* comprenant le polypeptide de fusion codé par l'acide nucléique de la revendication 1.

18. Procédé de production d'une particule chimérique de virus *Bamboo mosai'c* comprenant
la fourniture d'un acide nucléique de la revendication 1, l'acide nucléique ayant des séquences des cadres de lecture ouverte 1-4 du virus *Bamboo mosai'c ;*
la mise en contact de l'acide nucléique avec une feuille d'une plante hôte ;
le maintien de la feuille dans des conditions permettant la formation d'une particule virale ayant les polypeptides codés par l'acide nucléique, et
la purification de la particule virale à partir de la feuille.

19. Composition immunogène comprenant le polypeptide de fusion de la revendication 10.

20. Composition immunogène comprenant la particule de virus *Bamboo mosai'c* de la revendication 17.

21. Utilisation du polypeptide de fusion de la revendication 10 pour la fabrication d'une composition pour induire une réponse immune chez un sujet.

22. Utilisation selon la revendication 21, où le sujet est un animal non-humain.

23. Utilisation selon la revendication 22, où l'animal non-humain est un cochon.

24. Utilisation de la particule chimérique de virus *Bamboo mosai'c* de la revendication 17 pour la fabrication d'une composition pour induire une réponse immune chez un sujet.

25. Utilisation de la revendication 24, où le sujet est un animal non-humain.

26. Utilisation de la revendication 25, où l'animal non-humain est un cochon.


### SEQUENCE LISTING

<110> Academia Sinica
<120> RECOMBINANT VIRAL PROTEINS AND PARTICLES
<130> 08919-150EP1
<160> 14
<170> FastSEQ for Windows Version 4.0
<210> 1
   <211> 241
   <212> PRT
   <213> Bamboo mosaic virus
<400> 1
   Met Ser Gly Ala Gly Thr Gly Thr Gly Arg Gly Thr Gly Thr Gly Val
    1               5                  10                  15
   Gly Gly Thr Gly Gly Thr Gly Gly Thr Gly Gly Gly Gly Thr Gly Arg
               20                  25                  30
   Gly Gln Gln Ala Ala Ala Gln Pro Trp Glu Ala Ile Phe Thr Lys Asp
           35                  40                  45
   Asp Leu Ala Ala Ile Glu Pro Lys Pro Ala Ser Ala Asn Val Pro Asn
       50                  55                  60
   Thr Lys Gln Trp Ile Gly Ile Gln Ala Gly Leu Ile Lys Ala Gly Ala
   65                  70                  75                  80
   Thr Asp Ala Asn Phe Met Lys Val Leu Leu Gly Leu Ser Leu Glu Ala
                   85                  90                  95
   Phe Asp Arg Gly Ser Ser Glu Ala Thr Thr Trp Asp Gly Ile Thr Glu
               100                 105                 110
   Gly Val Glu His Arg Ala Ala Ala Asn Ala Ile Lys Glu Ala Asn Pro
           115                 120                 125
   Ile His Lys Val Thr Tyr Tyr Leu Ala Lys Pro Thr Phe Ala Ile Arg
       130                 135                 140
   Gln Ser Lys Asn Leu Pro Pro Ala Asn Phe Ala Lys Lys Asn Val Pro
   145                 150                 155                 160
   Ser Gln Tyr Lys Trp Cys Ala Phe Asp Ala Phe Asp Gly Leu Tyr Asp
                   165                 170                 175
   Pro Thr Cys Leu Ala Ser Glu Leu Pro Tyr Asp Ala Pro Ser Glu Ile
               180                 185                 190
   Asp Arg Met Ala Tyr Ala Thr Phe Lys Thr Ile Gln Ile Lys Ile Ala
           195                 200                 205
   Asn Asp Gln Lys Gly Phe Asn Leu Asn Tyr Asn Pro Asn Val Thr Gln
       210                 215                 220
   Ala Arg Leu Pro Asn Ala Pro Leu Pro Ala Leu Pro Glu Pro Thr Ser
   225                 230                 235                 240
   Asp
<210> 2
   <211> 724
   <212> DNA
   <213> Bamboo mosaic virus
<400> 2
   atgtctggag ctggaacggg aactgggcga gggaccggga ccggagtagg aggcactggg 60
   ggcacaggtg gcacaggcgg cggaggaaca ggtagagggc aacaagctgc agcccagccc 120
   tgggaggaat ttttactaag gacgacctgg ccgcaatcga gccaaaacct gcttcggcaa 180
   atgttccaaa cactaagcag tggatcggca ttcaagctgg actcatcaag gccggagcca 240
   cggacgcaaa cttctgaaag tactgctcgg cctcagtctc gaagctttcg acaggggctc 300
   atcagaagcc accacttggg atggaattac tgagggcgtg gagcaccgtg cagcagccaa 360
   cgccatcaag gaggcgaact gccaatacac aaggtcacct actacctagc caaaccgacg 420
   ttcgccatta gacaatcgaa aaacctcccc ccagcgaact tcgcaaagaa gaatgtgcca 480
   tcacaatata aatggtgtgc gttcgatgct ttgatggcct gtacgatcct acctgccttg 540
   cctcagaact accctacgac gccccctcag aaatagaccg aatggcgtac gctaccttca 600
   aaactataca gatcaagatc gccaatgacc agaaaggttc aacctcaact acaaccctaa 660
   cgtcacccag gctcgactcc ccaacgcgcc cctaccagct cttcccgaac caacatcaga 720
   ctaa                                                              724
<210> 3
   <211> 213
   <212> PRT
   <213> Foot-and-mouth disease virus
<400> 3
   Thr Thr Ser Ala Gly Glu Ser Ala Asp Pro Val Thr Ala Thr Val Glu
    1               5                  10                  15
   Asn Tyr Gly Gly Glu Thr Gln Val Gln Arg Arg Gln His Thr Asp Ser
               20                  25                  30
   Ala Phe Ile Leu Asp Arg Phe Val Lys Val Lys Pro Lys Glu Gln Val
           35                  40                  45
   Asn Val Leu Asp Leu Met Gln Ile Pro Ala His Thr Leu Val Gly Ala
       50                  55                  60
   Leu Leu Arg Thr Ala Thr Tyr Tyr Phe Ser Asp Leu Glu Leu Ala Val
   65                  70                  75                  80
   Lys His Glu Gly Asp Leu Thr Trp Val Pro Asn Gly Ala Pro Glu Thr
                   85                  90                  95
   Ala Leu Asp Asn Thr Thr Asn Pro Thr Ala Tyr His Lys Glu Pro Leu
               100                 105                 110
   Thr Arg Leu Ala Leu Pro Tyr Thr Ala Pro His Arg Val Leu Ala Thr
           115                 120                 125
   Val Tyr Asn Gly Ser Ser Lys Tyr Gly Asp Thr Ser Thr Asn Asn Val
       130                 135                 140
   Arg Gly Asp Leu Gln Val Leu Ala Gln Lys Ala Glu Arg Thr Leu Pro
   145                 150                 155                 160
   Thr Ser Phe Asn Phe Gly Ala Ile Lys Ala Thr Arg Val Thr Glu Leu
                   165                 170                 175
   Leu Tyr Arg Met Lys Arg Ala Glu Thr Tyr Cys Pro Arg Pro Leu Leu
               180                 185                 190
   Ala Ile Gln Pro Ser Asp Ala Arg His Lys Gln Arg Ile Val Ala Pro
           195                 200                 205
   Ala Lys Gln Leu Leu
       210
<210> 4
   <211> 639
   <212> DNA
   <213> Foot-and-mouth disease virus
<400> 4
   accacctctg cgggtgagtc tgcggacccc gtgactgcca ccgtcgagaa ctacggtggt 60
   gagacacaag tccagaggcg ccagcacacg gacagtgcgt tcatactgga caggttcgtg 120
   aaagtcaagc caaaggaaca agttaatgtg ttggacctga tgcagatccc tgcccacacc 180
   ttggtagggg cgctcctgcg aacggccacc tactacttct ctgacctgga gctggccgtc 240
   aagcacgagg gcgatctcac ctgggtccca aacggcgccc ctgagacagc actggacaac 300
   actaccaacc caacagctta ccacaaggaa cccctcacac ggctggcgct gccttacacg 360
   gctccacacc gtgtcttagc gaccgtctac aacgggagca gtaagtacgg tgacaccagc 420
   actaacaacg tgagaggtga ccttcaagtg ttagctcaga aggcagaaag aactctgcct 480
   acctccttca acttcggtgc catcaaggca actcgtgtta ctgaactact ctacagaatg 540
   aagagagccg agacatactg tcccaggccc cttctcgcca ttcaaccgag tgacgctaga 600
   cacaagcaga ggattgtggc acccgcaaaa cagcttctg                        639
<210> 5
   <211> 37
   <212> PRT
   <213> Foot-and-mouth disease virus
<400> 5
   Thr Val Tyr Asn Gly Ser Ser Lys Tyr Gly Asp Thr Ser Thr Asn Asn
    1               5                   10                  15
   Val Arg Gly Asp Leu Gln Val Leu Ala Gln Lys Ala Glu Arg Thr Leu
               20                  25                  30
   Pro Thr Ser Phe Asn
           35
<210> 6
   <211> 111
   <212> DNA
   <213> Foot-and-mouth disease virus
<400> 6
   accgtctaca acgggagcag taagtacggt gacaccagca ctaacaacgt gagaggtgac 60
   cttcaagtgt tagctcagaa ggcagaaaga actctgccta cctccttcaa c          111
<210> 7
   <211> 206
   <212> PRT
   <213> Bamboo mosaic virus
<400> 7
   Ala Ala Ala Gln Pro Trp Glu Ala Ile Phe Thr Lys Asp Asp Leu Ala
    1               5                   10                  15
   Ala Ile Glu Pro Lys Pro Ala Ser Ala Asn Val Pro Asn Thr Lys Gln
               20                  25                  30
   Trp Ile Gly Ile Gln Ala Gly Leu Ile Lys Ala Gly Ala Thr Asp Ala
           35                  40                  45
   Asn Phe Met Lys Val Leu Leu Gly Leu Ser Leu Glu Ala Phe Asp Arg
       50                  55                  60
   Gly Ser Ser Glu Ala Thr Thr Trp Asp Gly Ile Thr Glu Gly Val Glu
   65                  70                  75                  80
   His Arg Ala Ala Ala Asn Ala Ile Lys Glu Ala Asn Pro Ile His Lys
                   85                  90                  95
   Val Thr Tyr Tyr Leu Ala Lys Pro Thr Phe Ala Ile Arg Gln Ser Lys
               100                 105                 110
   Asn Leu Pro Pro Ala Asn Phe Ala Lys Lys Asn Val Pro Ser Gln Tyr
           115                 120                 125
   Lys Trp Cys Ala Phe Asp Ala Phe Asp Gly Leu Tyr Asp Pro Thr Cys
       130                 135                 140
   Leu Ala Ser Glu Leu Pro Tyr Asp Ala Pro Ser Glu Ile Asp Arg Met
   145                 150                 155                 160
   Ala Tyr Ala Thr Phe Lys Thr Ile Gln Ile Lys Ile Ala Asn Asp Gln
                   165                 170                 175
   Lys Gly Phe Asn Leu Asn Tyr Asn Pro Asn Val Thr Gln Ala Arg Leu
               180                 185                 190
   Pro Asn Ala Pro Leu Pro Ala Leu Pro Glu Pro Thr Ser Asp
   195 200 205
<210> 8
   <211> 620
   <212> DNA
   <213> Bamboo mosaic virus
<400> 8
   gcggccgcac agccctggga ggcaattttt actaaggacg acctggccgc aatcgagcca 60
   aaacctgctt cggcaaatgt tccaaacact aagcagtgga tcggcattca agctggactc 120
   atcaaggcgg agccacggac gcaaacttca tgaaagtact gctcggcctc agtctcgaag 180
   ctttcgacag gggctcatca gaagccacca cttgggatgg aattactgag ggcgtggagc 240
   accgtgcagc agccacgcca tcaaggaggc gaactgccca atacacaagg tcacctacta 300
   cctagccaaa ccgacgttcg ccattagaca atcgaaaaac ctccccccag cgaacttcgc 360
   aaagaagaat gtgccatcac atataaatgg tgtgcgttcg atgcctttga tggcctgtac 420
   gatcctacct gccttgcctc agaactaccc tacgacgccc cctcagaaat agaccgaatg 480
   gcgtacgcta ccttcaaaac tatacagaca agatcgccaa tgaccagaaa gggttcaacc 540
   tcaactacaa ccctaacgtc acccaggctc gactccccaa cgcgccccta ccagctcttc 600
   ccgaaccaac atcagactaa                                             620
<210> 9
   <211> 245
   <212> PRT
   <213> Artificial Sequence
<220>
   <223> Syntheticaly generated peptide
<400> 9
   Met Asp Thr Val Tyr Asn Gly Ser Ser Lys Tyr Gly Asp Thr Ser Thr
    1               5                  10                  15
   Asn Asn Val Arg Gly Asp Leu Gln Val Leu Ala Gln Lys Ala Glu Arg
               20                  25                  30
   Thr Leu Pro Thr Ser Phe Asn Ala Ala Ala Gln Pro Trp Glu Ala Ile
           35                  40                  45
   Phe Thr Lys Asp Asp Leu Ala Ala Ile Glu Pro Lys Pro Ala Ser Ala
       50                  55                  60
   Asn Val Pro Asn Thr Lys Gln Trp Ile Gly Ile Gln Ala Gly Leu Ile
   65                  70                  75                  80
   Lys Ala Gly Ala Thr Asp Ala Asn Phe Met Lys Val Leu Leu Gly Leu
                   85                  90                  95
   Ser Leu Glu Ala Phe Asp Arg Gly Ser Ser Glu Ala Thr Thr Trp Asp
               100                 105                 110
   Gly Ile Thr Glu Gly Val Glu His Arg Ala Ala Ala Asn Ala Ile Glu
           115                 120                 125
   Ala Asn Cys Pro Ile His Lys Val Thr Tyr Tyr Leu Ala Lys Pro Thr
       130                 135                 140
   Phe Ala Ile Arg Gln Ser Lys Asn Leu Pro Pro Ala Asn Phe Ala Lys
   145                 150                 155                 160
   Lys Asn Val Pro Ser Gln Tyr Lys Trp Cys Ala Phe Asp Ala Phe Asp
                   165                 170                 175
   Gly Leu Tyr Asp Pro Thr Cys Leu Ala Ser Glu Leu Pro Tyr Asp Ala
               180                 185                 190
   Pro Ser Glu Ile Asp Arg Met Ala Tyr Ala Thr Phe Lys Thr Ile Gln
           195                 200                 205
   Ile Lys Ile Ala Asn Asp Gln Lys Gly Phe Asn Leu Asn Tyr Asn Pro
       210                 215                 220
   Asn Val Thr Gln Ala Arg Leu Pro Asn Ala Pro Leu Pro Ala Leu Pro
   225                 230                 235                 240
   Glu Pro Thr Ser Asp
                   245
<210> 10
   <211> 736
   <212> DNA
   <213> Artificial Sequence
<220>
   <223> Syntheticaly generated oligonucleotide
<400> 10
   atggacaccg tctacaacgg gagcagtaag tacggtgaca ccagcactaa caacgtgaga 60
   ggtgaccttc aagtgttagc tcagaaggca gaaagaactc tgcctacctc cttcaacgcg 120
   gccgcacgcc ctgggaggca atttttacta aggacgacct ggccgcaatc gagccaaaac 180
   ctgcttcggc aaatgttcca aacactaagc agtggatcgg cattcaagct ggactcatca 240
   aggccggagc cacgacgcaa acttcatgaa agtactgctc ggcctcagtc tcgaagcttt 300
   cgacaggggc tcatcagaag ccaccacttg ggatggaatt actgagggcg tggagcaccg 360
   tgcagcagcc aacgccatca agaggcgaac tgcccaatac acaaggtcac ctactaccta 420
   gccaaaccga cgttcgccat tagacaatcg aaaaacctcc ccccagcgaa cttcgcaaag 480
   aagaatgtgc catcacaata taaatggttg cgttcgatgc ctttgatggc ctgtacgatc 540
   ctacctgcct tgcctcagaa ctaccctacg acgccccctc agaaatagac cgaatggcgt 600
   acgctacctt caaaactata cagatcaaga tcgccatgac cagaaagggt tcaacctcaa 660
   ctacaaccct aacgtcaccc aggctcgact ccccaacgcg cccctaccag ctcttcccga 720
   accaacatca gactaa                                                 736
<210> 11
   <211> 34
   <212> DNA
   <213> Artificial Sequence
<220>
   <223> Primer
<400> 11
   gggctagcac catggacacc gtctacaacg ggag 34
<210> 12
   <211> 26
   <212> DNA
   <213> Artificial Sequence
<220>
   <223> Primer
<400> 12
   ttgcggccgc gttgaaggag gtaggc 26
<210> 13
   <211> 24
   <212> DNA
   <213> Artificial Sequence
<220>
   <223> Primer
<400> 13
   gctctgggaa actgaatgac ttcg 24
<210> 14
   <211> 27
   <212> DNA
   <213> Artificial Sequence
<220>
   <223> Primer
<400> 14
   gacttctctt ccgctttctt aggttag 27